**Europäisches Patentamt**

**European Patent Office**

**Office européen des brevets**

(11) Veröffentlichungsnummer: **0 093 315**

**A2**

## (12) EUROPÄISCHE PATENTANMELDUNG

(21) Anmeldenummer: 83103795.7

(22) Anmeldetag: 19.04.83

(51) Int. Cl.³: **A 61 F 15/00**
**A 45 D 44/00**

(30) Priorität: 30.04.82 DE 3216264
30.04.82 DE 8212539 U

(43) Veröffentlichungstag der Anmeldung:
09.11.83 Patentblatt 83/45

(84) Benannte Vertragsstaaten:
AT BE CH DE FR GB IT LI LU NL SE

(71) Anmelder: Roescheisen GmbH & Co.
Scheffeltgasse 3
D-7900 Ulm(DE)

(72) Erfinder: Loos, Fred
Scheffeltgasse 3
D-7900 Ulm(DE)

(74) Vertreter: Patentanwälte Grünecker, Dr. Kinkeldey, Dr.
Stockmair, Dr. Schumann, Jakob, Dr. Bezold, Meister,
Hilgers, Dr. Meyer-Plath
Maximilianstrasse 58
D-8000 München 22(DE)

(54) Vorrichtung zum Spenden von Wattekugeln.

(57) Die vorliegende Erfindung betrifft eine Vorrichtung zum Spenden von Wattekugeln mit einem zweigeteilten Aufnahmebehälter für die Wattekugeln, dessen erster Teil mit einer durch Stege teilverschlossenen Durchlaßöffnung und dessen zweiter Teil mit einer Druckfläche bzw. einem Boden des Aufnahmebehälters versehen ist, wobei die Wattekugeln mittels einer Relativbewegung der beiden Teile des Aufnahmebehälters durch die Durchlaßöffnung hindurch bewegt werden. Es ist Aufgabe der Erfindung, eine Vorichtung zum Spenden von Wattekugeln der eingangs erwähnten Art so weiterzubilden, daß bei reduziertem Konstruktionsaufwand ein einwandfreies Spenden der Wattekugeln ohne Zerfasern und Verfilzen ermöglicht und gleichzeitig die Bedienbarkeit der Vorrichtung allgemein verbessert wird. Dies wird erfindungsgemäß dadurch erreicht, daß die Relativbewegung der beiden Teile des Aufnahmebehälters durch die Schwerkraft erreicht wird und der Durchmesser der Stege in der Ebene der Durchlaßöffnung im wesentlichen dem Radius der Wattekugeln entspricht.

Fig.1

## Beschreibung

Die vorliegende Erfindung betrifft eine Vorrichtung zum Spenden von Wattekugeln mit einem zweigeteilten Aufnahmebehälter für die Wattekugeln, dessen erster Teil mit einer durch Stege teilverschlossenen Durchlaßöffnung und dessen zweiter Teil mit einer Druckfläche bzw. einem Boden des Aufnahmebehälters versehen ist, wobei die Wattekugeln mittels einer Relativbewegung der beiden Teile des Aufnahmebehälters durch die Durchlaßöffnung hindurch bewegt werden.

Die in der Praxis benutzten Vorrichtungen zum Spenden von Wattekugeln bestehen im wesentlichen aus einem zweigeteilten Aufnahmebehälter, wobei der eine Teil als ein die Wattekugeln aufnehmender Vierkantrohr-Abschnitt ausgebildet ist, dessen Bodenöffnung durch eine den zweiten Teil darstellende lose Bodenplatte mit Feder und Abstützplatte beweglich verschließbar ist. Diese Bodenplatte drückt infolge der Kraftwirkung der unterhalb an ihr angebrachten druckbelasteten Schraubenfeder die Wattekugeln in Richtung der als teilverschlossene Durchlaßöffnung ausgebildeten oberen Öffnung des Vierkantrohr-Abschnittes. Dabei stützt sich die Feder über eine unterhalb angeordnete Abstützplatte an Vorsprüngen ab, die an den Innenwandungen des Vierkantrohr-Abschnittes nahe dessen unteren Abschlußkanten fest angebracht sind.

Diese feste Anbringung der Vorsprünge erfordert ein Kippen der losen Bodenplatte und der Abstützplatte in Längsrichtung des Vierkantrohr-Abschnittes, wenn beide Platten entfernt werden sollen. Dies ist beispielsweise zum Zwecke des Nachfüllens des Aufnahmebehälters mit Wattekugeln notwendig. Der Kippvorgang wird ermöglicht durch geringe Dickenabmessungen der beiden Platten und durch das Fehlen einer Führung für die Feder.

Die bisher beschriebene Vorrichtung weist mehrere Nachteile auf. Der komplizierte Aufbau, insbesondere des zweiten Teiles des Aufnahmebehälters, bedingt einen unerwünscht hohen Kostenaufwand. Das Nachfüllen des Aufnahmebehälters läßt sich nur in umständlicher Art und Weise durchführen. Dies wird besonders dadurch erschwert, daß die Feder drehbar mit der losen Bodenplatte und der Abstützplatte verbunden ist, so daß nach Herausnahme aus dem Vierkantrohr-Abschnitt beide Platten sich gegeneinander verdrehen können. Dies erschwert das Einsetzen zurück in die Arbeitsposition. Außerdem ist es leicht möglich, daß die lose Bodenplatte und die Arbeitsplatte während der Entnahme aus dem Vierkantrohr-Abschnitt infolge der Feder wegspringen und verlorengehen.

Das Fehlen der Führung für die Feder sowie die Konstruktion der Feder selbst bedingt eine außerzentrische Kraftwirkung, so daß die lose Bodenplatte einen ungleichmäßigen Druck auf die Wattekugeln ausübt. Weiterhin ist es, bedingt durch die Konstruktion der Vorrichtung, nicht möglich, bei bestimmungsgemäßer Benutzung den Druck auf die Wattekugeln individuell zu verändern. Nur durch Hochheben der Vorrichtung wird der Zugang zur Bodenöffnung freigegeben, so daß der Benutzer über die Abstützplatte und die Feder einen erhöhten Druck auf die lose Bodenplatte und somit auf die Wattekugeln ausüben kann. Dabei macht sich die konstruktiv bedingte Kippmöglichkeit der Platte störend bemerkbar. Insbesondere bei gerginggefülltem Aufnahmebehälter kippt die Abstützplatte seitlich weg und macht eine Druckerhöhung auf die Wattekugeln unmöglich.

Die Durchlaßöffnungen der in der Praxis benutzten Vorrichtungen zum Spenden von Wattekugeln werden durch gitterförmig angeordnete Stege mit im wesentlichen kreisförmigem Querschnitt teilverschlossen, die jeweils an beiden Stegenden eingespannt sind. Der Durchmesser dieser Stege steht zu dem Durchmesser der Wattekugeln in einem Verhältnis zu ca. 1 : 8. Dies ist insofern nachteilig, als die gegen die Stege gedrückten Wattekugeln sich nicht an diesen entlang nach außen abwälzen können, sondern sich an ihnen verfangen. Das heißt, die Stege drücken sich infolge ihres geringen Durchmessers in die Wattekugeln hinein und halten diese in einem unerwünscht hohen Maße zurück. Sie wirken sozusagen als Teiler der Wattekugeln, wodurch diese ihre kugelförmige Gestalt verlieren und auseinanderfasern. Dadurch wird eine Verfilzung der Wattekugeln begünstigt und die Entnahme derselben aus dem Aufnahmebehälter erschwert. Die gitterförmige Anordnung der Stege ist ungünstig, da die Kreuzungspunkte der Stege quasi als Sperre für die Wattekugeln wirken.

Es sind auch Vorrichtungen zum Spenden von Wattekugeln bekannt, bei denen die die Durchlaßöffnung teilverschließenden Stege parallel zueinander und nicht in Gitterform angeordnet sind. Allerdings weisen diese Stege einen flachen Querschnitt mit einer Breite auf, die etwa dem Durchmesser der Wattekugeln entspricht.

Die Stege wirken somit als ausgesprochene Rückhaltemittel für die Wattekugeln. Außerdem handelt es sich hier um einteilige Vorrichtungen, bei denen kein Druck auf die Wattekugeln ausgeübt werden kann. Ein Durchtreten derselben durch die Durchlaßöffnung ist somit nicht möglich.

Es ist Aufgabe der Erfindung, eine Vorrichtung zum Spenden von Wattekugeln der eingangs erwähnten Art so weiterzubilden, daß bei reduziertem Konstruktionsaufwand ein einwandfreies Spenden der Wattekugeln ohne Zerfasern und Verfilzen ermöglicht und gleichzeitig die Bedienbarkeit der Vorrichtung allgemein verbessert wird.

Dies wird erfindungsgemäß dadurch erreicht, daß die Relativbewegung der beiden Teile des Aufnahmebehälters durch die Schwerkraft erreicht wird und der Durchmesser der Stege in der Ebene der Durchlassöffnung im wesentlichen dem Radius der Wattekugeln entspricht.

Durch Benutzung der Schwerkraft zur Bewegung der beiden Teile des Aufnahmebehälters relativ zueinander wird eine Reduzierung des konstruktiven Aufwandes erreicht. Eine in Richtung der Durchlaßöffnung drückende Feder, eine Abstützplatte sowie als Gegenlager dienende Vorsprünge an den Innenwandungen des die Wattekugeln aufnehmenden Teiles des Aufnahmebehälters sind nicht mehr erforderlich. Ebenso entfallen die an der losen Bodenplatte sowie an der Abstützplatte angebrachten Verbindungselemente für die Feder.

Gleichzeitig werden dadurch sämtliche Voraussetzungen geschaffen, um das Nachfüllen zu erleichtern, einen gleichmäßigen Druck auf die Wattekugeln zu erzielen und die Bedienbarkeit allgemein zu verbessern. Insbesondere ist es möglich, während der Benutzung der Vorrichtung den Druck auf die Wattekugeln individuell zu vergrößern. Bei einem zweigeteilten Aufnahmebehälter wird der obere Teil durch die Schwerkraft nach unten bewegt. In der einfachsten Ausführung ist dieses obere Teil frei zugänglich und kann somit durch den Benutzer in beliebigem Maße nach unten gedrückt werden. Dadurch wird eine individuelle Druckerhöhung auf die Wattekugeln erreicht. Auch ist eine permanente Druckveränderung möglich, die auch außerhalb der Benutzungsdauer erhalten bleibt. Dazu werden beispielsweise auswechselbare Gewichte an dem oberen Teil des Aufnahmebehälters angebracht.

Durch Verwendung der Stege mit einer im wesentlichen dem Radius der Wattekugeln entsprechenden Durchmesser wird das Hindurchtreten der Wattekugeln durch die Durchlaßöffnung erleichtert. Die Stege wirken hier nicht als Rückhaltemittel und als Teiler, sondern als Führungsmittel für die Wattekugeln. Es besteht keine Gefahr des Zerfaserns und des Verfilzens der Wattekugeln. Eine diesbezüglich unterstützende Maßnahme stellt eine parallele Anordnung der Stege dar.

Es ist vorteilhaft, den die Durchlaßöffnung für die Wattekugeln aufweisenden ersten Teil des Aufnahmebehälters als oberen Teil mit einer der Durchlaßöffnung gegenüberliegenden Bodenöffnung zu versehen und insgesamt auf dem als Stempel ausgebildeten zweiten, unteren Teil des Aufnahmebehälters vertikal verschiebbar anzuordnen.

Dies bedeutet, daß die obere Begrenzungsfläche des oberen Teiles des Aufnahmebehälters als Durchlaßöffnung für die Wattekugeln ausgebildet ist. Der als Stempel ausgestaltete untere Teil dringt während der Abwärtsbewegung des oberen Teiles in diesen ein und drückt die Wattekugeln in einer geraden Bahn in Richtung der Durchlaßöffnung und durch dieselbe hindurch. Im Gegensatz zu einer seitlich angeordneten Durchlaßöffnung wird hierdurch bei geringerem Kraftaufwand bzw. bei geringerem Gewicht des oberen Teiles ein verbessertes Durchtreten der Wattekugeln durch die Durchlaßöffnung und eine größere Stabilität während des Entnehmens der Wattekugeln erzielt. Die vertikale Bewegung des oberen Teiles wirkt sich in dieser Hinsicht besonders günstig aus. Weiterhin erleichtert die soeben beschriebene Ausbildung des Aufnahmebehälters das Nachfüllen desselben mit Wattekugeln. Der obere Teil des Aufnahmebehälters wird lediglich von dem unteren Teil abgehoben, gewendet und durch die nach oben offene Bodenöffnung hindurch mit Wattekugeln gefüllt. In dieser Stellung kann der als Stempel ausgebildete Teil aufgesetzt und anschließend der Aufnahmebehälter insgesamt wieder zurück in seine Benutzungsstellung gewendet werden.

Zur weiteren Verbesserung des Hindurchtretens der Wattekugeln durch die Durchlaßöffnung ist es vorteilhaft, zumindest einen Teil der Stege elastisch auszubilden. Hierbei sind die elastischen Stege vorzugsweise mit jeweils wenigstens einem Stegende gegenüber den durchtretenden Wattekugeln elastisch ausweichend in wenigstens einer die Stegachse einschließenden Ebene gelagert.

Der dem Stand der Technik gemeinsame Nachteil, daß der gegenseitige Abstand der Stege und deren Durchmesser in einem bestimmten Verhältnis zueinander stehen und die Vorrichtung infolgedessen nur für Wattekugeln eines bestimmten Durchmessers verwendet werden kann, wird hierdurch überwunden.

Wenigstens eines der beiden Stegende jedes elastischen Steges ist in einer oder mehreren sich entlang der Stegachse erstreckenden Ebenen, beispielsweise der Ebene der Durchlaßöffnung oder einer dazu normalen Ebene oder einer aus beiden Ebenen resultierenden Ebene frei beweglich. Demzufolge kann der jeweilige Steg während der Entnahme einer Wattekugel derselben ausweichen, wobei der Abstand zum benachbarten Steg in Abhängigkeit vom Durchmesser der Wattekugel vergrößert wird. Genauer gesagt, entspricht die Abstandsvergrößerung ohne Berücksichtigung von Formänderungen der Wattekugel infolge der elastischen Kräfte im wesentlichen der Differenz zwischen dem Wattekugeldurchmesser und dem ursprünglichen Stegabstand im Ruhezustand. Auch unterschiedlich große Wattekugeln können nun ungeachtet der Stegabstände leicht aus ein und demselben Behälter entnommen werden, da der jeweilige Stege so weit ausweicht, daß die Wattekugel durch den vergrößerten Abstand zum benachbarten Steg ungehindert hindurchtreten kann. Lediglich eine Abstimmung des Stegabstandes im Ruhezustand mit dem Durchmesser der kleinsten Wattekugel ist erforderlich, um ein ausreichendes Zurückhalten der Wattekugeln während deren Nicht-Entnahme zu gewährleisten. Die Ausweichbewegung der Stege ist nicht nur auf einen mittleren Stegabschnitt beschränkt, sondern erstreckt sich über nahezu die gesamte Steglänge. Die Elastizität der Stege be-

wirkt, daß einerseits die Wattekugeln während der Nicht-
Entnahme in ausreichendem Maße zurückgehalten werden
und andererseits die Stege nach der Entnahme der Wattekugeln ohne Verwendung zusätzlicher Hilfsmittel wie
beispielsweise Druckfedern automatisch in die Ruhestellung zurückkehren. Die Elastizität wird so gewählt, daß
ein übermäßiges Zusammendrücken der Wattekugeln vermieden wird.

Um jeden elastischen Steg an beiden Stegenden beweglich
zu gestalten und ein automatisches Zurückkehren in die
Ruhestellung zu gewährleisten, werden die elastischen
Stege an beiden Stegenden in jeweils einer sich entlang der Stegachse erstreckenden Ebene gegenüber den
durchtretenden Wattekugeln ausweichend gelagert, wobei
die eine Ebene gegenüber der anderen um etwa 90° gedreht ist.

Gemäß einer Weiterbildung der Erfindung werden die
elastischen Stege an einem Stegende in der Art eines
Freiträgers fest gelagert und an dem anderen Stegende
in einer sich entlang der Stegachse erstreckenden Ebene
gegenüber den durchtretenden Wattekugeln ausweichend gelagert. Diese Ebene kann mit der Ebene der Durchlaßöffnung für die Wattekugeln oder einer dazu parallelen
Ebene übereinstimmen, kann sich aber auch dazu normal
erstrecken oder eine aus beiden Ebenen resultierende
Ebene darstellen.

Die Entnahme der Walttekugeln wird dadurch erleichtert,
daß zumindest die elastischen Stege einen im wesentlichen
kreisförmigen Querschnitt aufweisen. Eine weitere Verbesserung in dieser Hinsicht ergibt sich dadurch, daß zumindest die elastischen Stege um ihre Stegachse drehbar sind.

Eine einfache konstruktive Ausführung der Erfindung
verwendet elastische Stege, die in der Art eines Freiträgers an lediglich einem Stegende fest gelagert sind.
Das andere Stegende ist frei und die Ausweichbewegung
kann je nach Angriffspunkt der Wattekugel in jeder
sich entlang der Stegachse erstreckenden Ebene erfolgen.

Eine weitere konstruktive Vereinfachung ergibt sich
dann, wenn zumindest die elastischen Stege mit ihrem
jeweils in der Art eines Freiträgers gehaltenen Stegende einteilig mit dem ersten Teil des Aufnahmebehälters
verbunden sind.

Eine Beschränkung der Ausweichbewegung auf eine Ebene
kann in einfacher Weise dadurch erreicht werden, daß
zumindest die elastischen Stege einen rechteckigen Querschnitt aufweisen, dessen Breitseite in die Ausweichrichtung der Stege weist. Eine Beschränkung auf die
Ebene der Durchlaßöffnung oder einer dazu parallelen
Ebene beispielsweise ist dann vorteilhaft, wenn die
Stege sich einander kreuzend in zwei parallelen Ebenen
angeordnet sind. Eine Ausweichbewegung der Stege der dem
Behälterinneren zugewandten Ebene in Richtung der Stege
der anderen Ebene würde eine Abstandsverminderung zu denselben bedeuten und somit das Entnehmen der Wattekugeln
erschweren.

Um eine über die gesamte Länge der Stege im wesentlichen
gleichbleibende Abstandsvergrößerungen zwischen benachbarten elastischen Stegen erzielen zu können, werden dieselben vorzugsweise jeweils an entgegengesetzten Stegenden in der Art eines Freiträgers fest gelagert.

Gemäß einer Weiterbildung der Erfindung ist jeweils ein elastischer Steg abwechselnd mit einem weniger elastischen und/oder mit einem an beiden Stegenden fest gelagerten Steg angeordnet. Dadurch wird erreicht, daß bei Stegen, die in einer zur Durchlaßöffnung normalen Ebene ausweichend gelagert sind, lediglich einer von zwei benachbarten Stegen während der Entnahme einer Wattekugel ausweicht und somit den Abstand zum anderen Steg vergrößert. Ein gleichzeitiges Ausweichen zweier benachbarter Stege in einer zur Durchlaßöffnung normalen Ebene würde keine Abstandsvergrößerung bedeuten und somit für die Entnahme der Wattekugeln wirkungslos bleiben.

Vorzugsweise werden die Stege im wesentlichen parallel angeordnet. Hierbei ist es vorteilhaft, die Stege als gerade Stege mit glatter Oberfläche auszubilden. Die Stege können im wesentlichen in einer Ebene angeordnet sein. Um eine möglichst gleichmäßige Druckverteilung auf die Wattekugeln zu erreichen, wird die obere Begrenzungsfläche des als Stempel ausgebildeten zweiten Teiles des Aufnahmebehälters vorteilhafterweise als eine ebene und senkrecht zur Längsachse des Aufnahmebehälters angeordnete Stempelfläche gestaltet.

Vorzugsweise werden der erste, obere und der zweite Teil des Aufnahmebehälters zylindrisch ausgebildet. Zum einen erleichtert dies die Herstellung beider Teile und zum anderen wird dadurch ein Verdrehen des oberen Teiles gegenüber dem Stempel ermöglicht. Während der Benutzung der Vorrichtung durch Herunterdrücken des oberen Teiles wirkt sich das Verdrehen desselben gegenüber dem Stempel besonders günstig auf das Abwälzverhalten der Wattekugeln an den Oberflächen der Stege aus. Dadurch wird das Hindurchtreten derselben durch die Durchlaßöffnung verbessert.

Als Sicherheitsmaßnahme gegen unbeabsichtigtes Lösen des oberen Teiles des Aufnahmebehälters vom unteren Teil wird vorteilhafterweise der Stempel mit einem der Stempelfläche zugeordneten, durch mindestens eine im wesentlichen vertikale Nut durchbrochenen umlaufenden Bund versehen und der erste Teil des Aufnahmebehälters an seiner Innenwandung mit einem der Nut entsprechenden Vorsprung ausgestattet. Somit kann der erste Teil nur in einer bestimmten Relativposition zum Stempel auf diesen aufgesetzt bzw. von ihm gelöst werden. Die Gefahr des unbeabsichtigten Lösens wird durch gegenseitiges Verdrehen der beiden Teile reduziert.

Um diese Gefahr gänzlich auszuschalten, ist es vorteilhaft, daß die Nut eine geringere Tiefe aufweist als der Bundstärke entspricht und daß der der Nut entsprechende Vorsprung verstellbar, beispielsweise als Schraube, ausgebildet ist. Der erste, obere Teil des Aufnahmebehälters wird auf den Stempel aufgesetzt und soweit heruntergedrückt bis die Schraube sich unterhalb der Nut bzw. des Bundes befindet. Sodann wird die Schraube soweit in Richtung Stempel-Längsachse verstellt bis keine Eingriffsmöglichkeit mehr mit der Nut besteht.

Mittels mindestens eines im wesentlichen senkrecht an der Stempelfläche angebrachten Flügels kann durch Drehen des oberen Teiles des Aufnahmebehälters gegenüber dem Stempel ein Auflockern der zusammengedrückten Wattekugeln erreicht und dadurch die Gefahr einer Verfilzung
derselben vermieden werden.

Gemäß einer Weiterbildung der Erfindung weist der auf
der Stempelfläche angebrachte Flügel einen im wesentlichen dreieckigen, mit der Basis zur Stempelfläche
weisenden Querschnitt auf, so daß während des Drehens
des oberen Teils zum Zwecke des Auflockerns der Wattekugeln gleichzeitig ein Druck auf dieselben in Richtung
Durchlaßöffnung ausgeübt wird.

Um mit der gesamten Stempelfläche einen Druck auf die
Wattekugeln während des Drehens des oberen Teiles gegenüber der Stempelfläche ausüben zu können, ist es vorteilhaft, die obere Begrenzungsfläche des Flügels als
eine sich über die gesamte Stempelfläche erstreckende,
in Umfangsrichtung von der Oberkante bis zur Unterkante
des Flügels abfallende Schräge auszubilden. Je nach Drehrichtung des oberen Teiles werden die Wattekugeln aufgelockert oder in Richtung Durchlaßöffnung gedrückt.

Zweckmäßigerweise wird der Aufnahmebehälter insgesamt
von einem äußeren Behälter umschlossen, der mit einer
oberen Öffnung versehen ist, durch welche das die Durchlaßöffnung aufweisende obere Teil des Aufnahmebehälters
hindurchtreten kann. Die Verwendung dieses äußeren Behälters stellt insofern eine hygienische Maßnahme dar,
als dadurch eine glatte äußere Ummantelung mit geringer
Angriffsfläche für Verunreinigungen und leichterer Reinigungsmöglichkeit geschaffen wird.

Eine weitere hygienische Maßnahme stellt die Verwendung des ersten, oberen Teiles des Aufnahmebehälters als eine mit Wattekugeln gefüllte und mittels Folien an der Bodenöffnung und der Durchlaßöffnung luftdicht verschlossenen, auswechselbaren Einsatzpackung dar. Es ist nicht mehr erforderlich, die Wattekugeln aus einem größeren Vorratsbehälter zu entnehmen und in den Aufnahmebehälter umzufüllen. Vielmehr wird nach Entfernen der Folien die Einsatzpackung lediglich auf den Stempel aufgesetzt. Die Vorteile dieser Maßnahme sind bekannt.

Eine Weiterbildung sieht einen im wesentlichen als Trog gestalteten Aufnahmebehälter vor, der die Form eines Zylinder-Viertels aufweist. Die dem einen Zylinder-Radius folgende erste Behälterwandung ist hierbei als erfindungsgemäße Durchlaßöffnung und die dem anderen Zylinder-Radius folgende zweite Behälterwandung als verschwenkbare Klappe ausgebildet. Sie ist um eine parallel zu und nahe der Zylinderachse angebrachte Schwenkachse verschwenkbar angeordnet. Senkrecht zur Schwenkachse erstreckt sich diese Klappe bis zu der dritten, die Mantelfläche des Zylinder-Viertels darstellende Behälterwandung. Hierbei stellt die Klappe sozusagen das erste, obere Teil des Aufnahmebehälters dar, die ebenfalls wie dieser durch die Schwerkraft einen Druck auf die Wattekugeln in Richtung der Durchlaßöffnung ausübt.

Eine besonders vorteilhafte Ausbildung der Erfindung weist einen Aufnahmebehälter auf, der in mindestens zwei Kammern zur Aufnahme von Wattekugeln mit unterschiedlichen Durchmessern aufgeteilt ist.

Wird beispielsweise der erste Teil als Einsatz innerhalb des zweiten Teiles zum Zwecke der Entnahme der Wattekugeln ausgebildet, so kann derselbe lediglich aus den Stegen bestehen, die an ihren, den Führungsflächen des zweiten Teiles zugewandten Enden miteinander verbunden sind, wodurch die durch die Stege teilverschlossene Durchlaßöffnung gebildet wird. Diese Konstruktion ist einfach und materialsparend, wodurch eine Verringerung des Kostenaufwandes im Vergleich zum Stande der Technik erzielt wird. Darüber hinaus können die Herstellungskosten eines derart ausgebildeten ersten Teiles beispielsweise dadurch gesenkt werden, daß derselbe einteilig im Spritzgußverfahren, im Druckgußverfahren oder einem anderen Gießverfahren produziert wird.

Vorzugsweise wird der zweite Teil des Aufnahmebehälters als ein Behältnis für die Wattekugeln ausgebildet, dessen Wandungen zur Führung des ersten Teiles dienen. Außer der oberen Öffnung ist dieses Behältnis allseitig geschlossen, so daß es neben der einfachen, materialsparenden Form eine hohe Eigensteifigkeit aufweist. Es kann einteilig ausgebildet sein, jedoch es ist auch denkbar, den Boden dieses Behältnisses beispielsweise abnehmbar zu gestalten. Dadurch wird das Nachfüllen erleichtert. In jedem Fall ist eine kostengünstige Herstellung mittels des Spritzgußverfahrens beispielsweise möglich. Zum Schutze der Wattekugeln kann die obere Öffnung des Behältnisses mit einem Deckel verschließbar ausgebildet sein. Selbstverständlich kann der Boden des Behältnisses auch nach oben weisen, so daß die Wattekugeln nach unten durch die jetzt untere Öffnung austreten.

Gemäß einer Weiterbildung der Erfindung wird die obere Öffnung des zweiten Teiles des Aufnahmebehälters von dessen Führungsflächen bzw. Wandungen direkt begrenzt. Dadurch ist die Verwendung einer einfachen Dose beispielsweise möglich, die materialersparend und einfach herzustellen ist. Außerdem können die bekannten Vorteile von zugelieferten, handelsüblichen Behältnissen ausgenutzt werden.

Der als Einsatz ausgebildete erste Teil läßt sich aus einem solchen Behälter leicht herausnehmen, so daß ein einfaches Nachfüllen durch die obere Öffnung des Behältnisses hindurch erfolgen kann.

Eine besonders leichte Entnahme der Wattekugeln wird dadurch erreicht, daß die obere Öffnung des Behältnisses sowie der die Durchlaßöffnung aufweisende Einsatz schräg zur Längsachse des Behältnisses verlaufend ausgebildet sind. Durch Aufstellen des Behältnisses derart, daß seine obere Öffnung dem Benutzer zugewandt wird, kann dieser ohne Abwinkeln des Handgelenkes auch aus größerer seitlicher Entfernung die Wattekugeln entnehmen.

Hierbei ist es besonders vorteilhaft, das Behältnis und den Einsatz abweichend von der Zylinderform auszubilden, so daß ein Verdrehen des Einsatzes innerhalb des Behältnisses ausgeschlossen ist. Hierbei sind in der Draufsicht nicht nur oval oder eckig ausgebildete Körper denkbar, sondern es können auch zylindrische Körper mit wenigstens einer in Längsrichtung verlaufenden Nut-/Federanordnung beispielsweise verwendet werden.

Zur Führung der Wattekugeln zur Durchlaßöffnung während der Relativbewegung der beiden Teile des Aufnahmebehälters ist es vorteilhaft, den ersten Teil des Aufnahmebehälters mit Schrägflächen zu versehen, die in Richtung des Bodens und der Wandungen des zweiten Teiles des Aufnahmebehälters verlaufen. Eine solche Ausbildung ist besonders vorteilhaft, wenn der erste Teil des Aufnahmebehälters bei Betrachtung in der Draufsicht um so viel größer ist als seine Durchlaßöffnung, daß ein Stauraum entsteht, in dem Wattekugeln zurückgehalten und verdichtet werden.

Nachstehend wird die Erfindung anhand der Beschreibung von acht Ausführungsbeispielen unter Bezugnahme auf die entsprechenden Figuren näher beschrieben, wobei gleiche Bezugszeichen für analoge Bauteile verwendet werden.

Es zeigt:

Figur 1 eine perspektivische Darstellung eines ersten Ausführungsbeispieles der Erfindung,

Figur 2 eine Seitenansicht der in Figur 1 dargestellten Vorrichtung,

Figur 3 eine perspektivische Darstellung eines zweiten Ausführungsbeispieles der Erfindung,

Figur 4 eine Seitenansicht der in Figur 3 dargestellten Vorrichtung,

Figur 5 eine Schnittansicht eines dritten Ausführungsbeispieles der Erfindung

Figur 6 und 7 perspektivische Ansichten von mit Flügeln versehenen Stempelflächen des unteren Teiles von Aufnahmebehältern,

Figur 8   eine perspektivische Darstellung eines
          vierten Ausführungsbeispieles der Erfindung,

Figur 9   einen Vertikalschnitt durch die in Figur 8
          gezeigte Vorrichtung, jedoch zusätzlich mit
          einem Deckel,

Figur 10  eine perspektivische Darstellung eines fünften
          Ausführungsbeispiels der Erfindung,

Figur 11  einen Vertikalschnitt durch die in Figur 10
          gezeigte Vorrichtung.

Figur 12  eine perspektivische Darstellung eines sechsten
          Ausführungsbeispieles der Erfindung,

Figur 13  einen Vertikalschnitt durch die in Figur 12
          gezeigte Vorrichtung, jedoch zusätzlich mit
          einem Deckel, wobei die in Figur 12 nicht sichtbare, dem Betrachter zugewandte  Steglagerung
          bei Betrachtung vom Behälterinneren aus dargestellt ist,

Figur 14  eine weggebrochene Draufsicht von Figur 12,

Figur 15  eine weggebrochene Draufsicht eines siebten
          Ausführungsbeispieles der Erfindung,

Figur 16  einen Teilschnitt entlang der Linie V-V in
          Figur 15,

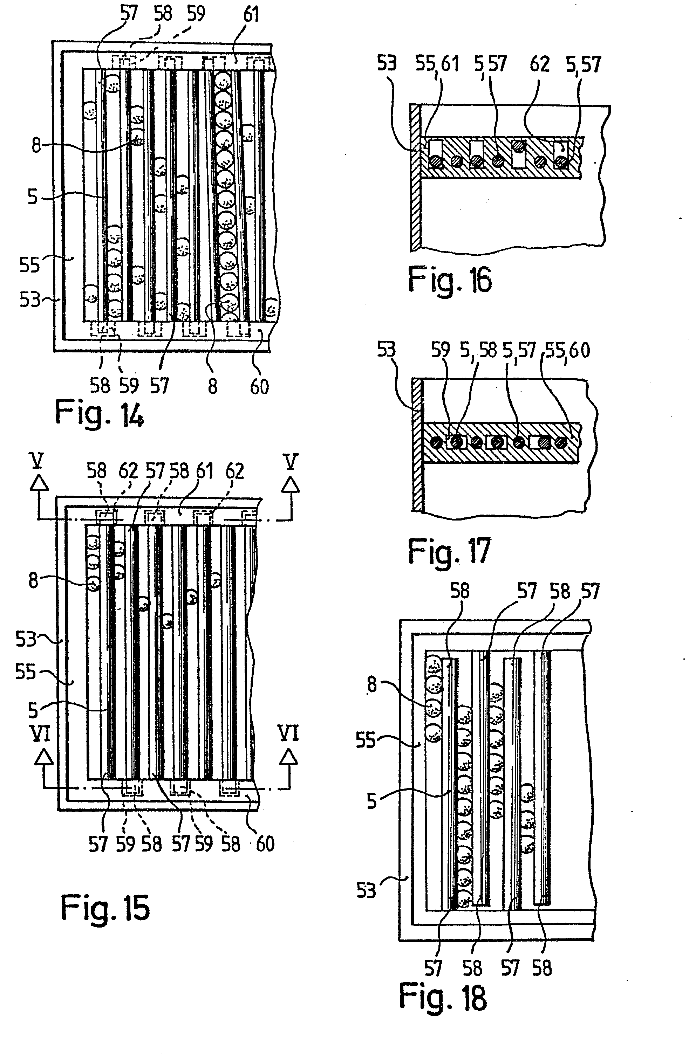

Figur 17  einen Teilschnitt entlang der Linie VI-VI
          in Figur 15,
          und

Figur 18  eine weggebrochene Draufsicht eines achten
Ausführungsbeispieles der Erfindung.

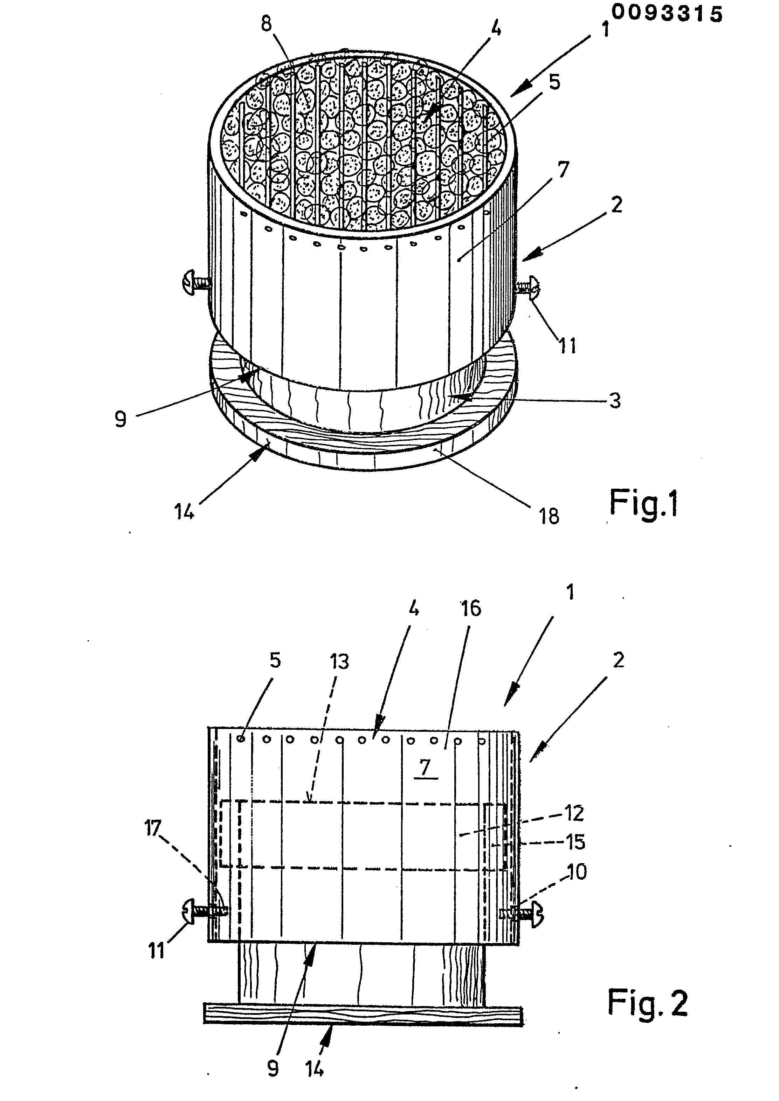

Die Figuren 1 und 2 zeigen als erstes Ausführungsbeispiel der Erfindung eine zylindrische Vorrichtung zum Spenden von Wattekugeln mit einem zweigeteilten Aufnahmebehälter 1, der im wesentlichen aus einem ersten, oberen Teil 2 und einem als Stempel 3 ausgebildeten zweiten, unteren Teil besteht. Der obere Teil 2 ist als geradzylindrischer Rohrabschnitt ausgebildet, dessen obere Öffnung, die hier als Durchlaßöffnung 4 bezeichnet werden soll, durch parallel angeordnete Stege 5 teilverschlossen ist. Die Aufnahme der Stegenden ist hier nur schematisch dargestellt und wird weiter unten detailliert beschrieben. Die Stege 5 sind elastisch ausgebildet und weisen einen kreisrunden Querschnitt auf, dessen Durchmesser im wesentlichen dem Radius der Wattekugeln 8 entspricht. Die Stege 5 können selbstverständlich auch jede andere Querschnittsform aufweisen. Sie können auch starr ausgebildet sein.

In der Wandung 7 des oberen Teiles 2 sind nahe seiner Bodenöffnung 9 zwei diametral gegenüberliegende, durchgehende Öffnungen 10 angeordnet, die je eine verstellbare Schraube 11 aufnehmen.

Der Stempel 3 ist als ein im wesentlichen geradzylindrischer Körper mit ebenen Begrenzungsflächen und mit geringerem Durchmesser als das obere Teil 2 ausgebildet. Ein umlaufender Bund 12 erstreckt sich von der oberen Begrenzungsfläche des Stempels 3, die hier als Stempelfläche 13 bezeichnet werden soll, entlang einem Teilstück des Stempels 3 in Richtung seiner unteren Begrenzungsfläche 14. Der Durchmesser des Bundes 12 steht zum Innendurchmesser des oberen Teiles 2 des Aufnahmebehälters 1 in einem solchen Verhältnis, daß ein verbleibendes Spiel eine leichtgängige Bewegung zwischen dem oberen Teil 2 und dem Stempel 3 erlaubt und gleichzeitig ein Einklemmen der Wattekugeln 8 zwischen diesen Teilen verhindert.

Der Bund 12 weist an zwei diametral gegenüberliegenden Stellen je eine vertikale durchgehende Nut 15 mit einer der Bundstärke entsprechenden Tiefe auf. Während des Abhebens bzw. des Aufsetzens des oberen Teiles 2 auf den Stempel 3, beispielsweise zum Füllen des Innenraumes 16 des oberen Teiles 2 mit den Wattekugeln 8, treten die in den Innenraum 16 hineinragenden Teile der Schraube 11 als Vorsprünge 17 durch die Nuten 15 hindurch. Die Nutbreite ist dementsprechend gewählt.

Um eine ausreichende Standsicherheit des Aufnahmebehälters 1 zu gewährleisten, ist die untere, als Aufstandsfläche dienende Begrenzungsfläche 14 des Stempels 3 durch einen umlaufenden Ansatz 18 entsprechend verbreitert.

Bei der Ausführungsform der Figuren 1 und 2 sind lediglich zwei Schrauben 11 angedeutet. Sofern das obere Teil gegenüber dem unteren Teil noch besser geführt sein soll, ist es möglich, drei gleichmäßig über den Umfang verteilte Schrauben vorzusehen. Mit diesen kann ein eventuell noch störendes Wackeln des Oberteils gegenüber dem Unterteil verändert werden.

Bezüglich der Schrauben ist es auch denkbar, dieselben durch einfache Handhabungselemente zu ersetzen, die beispielsweise unter Beaufschlagung einer Feder sich normalerweise in ihrer Arretierstellung befinden. Soll das Oberteil von Stempel 3 gelöst werden, so können diese
Elemente gegen die Kraft der Feder herausgezogen und
verschwenkt werden, worauf sie in dieser Stellung bis
zum Wiedereinsetzen des Oberteils auf dem Unterteil
und die erneute Verschwenkung verharren.

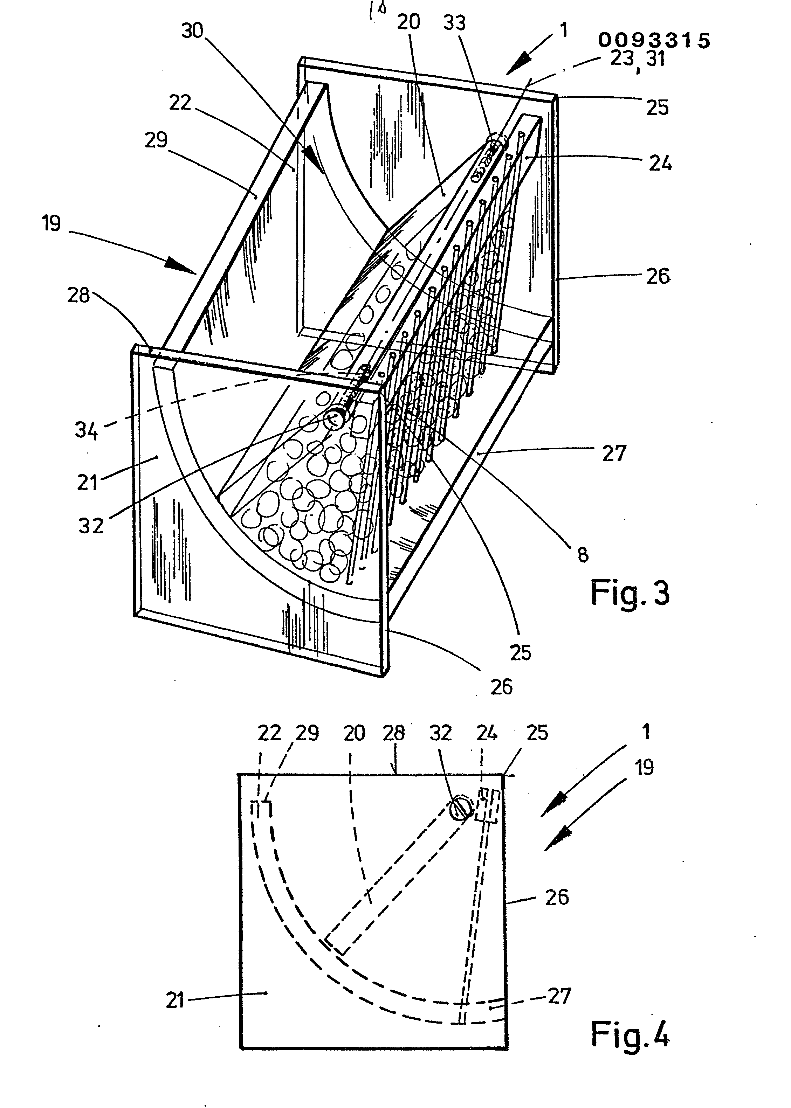

Anhand der Figuren 3 und 4 wird im folgenden als zweites
Ausführungsbeispiel der Erfindung eine Vorrichtung zum
Spenden von Wattekugeln mit einem zweigeteilten Aufnahmebehälter 1 in Form eines Troges 19 beschrieben.
Hierbei stellt der Trog 19 den ersten Teil und eine denselben beweglich verschließbare Klappe 20 den zweiten
Teil des Aufnahmebehälters 1 dar. Zwei rechteckige,
parallel zueinander mit Abstand angeordnete, vertikale
Platten 21 stellen die seitlichen Begrenzungsflächen
des Troges 19 dar. Eine die Mantelfläche eines Zylinder-
Viertels bildende gekrümmte Behälterwandung 22 verbindet die Platten 21, wobei die Zylinderachse 23 senkrecht zu denselben angeordnet ist. Eine zweite Verbindung der Platten 21 wird durch einen senkrecht zu denselben verlaufenden, im Querschnitt rechteckigen Querstab 24 gebildet, der nahe den der Zylinderachse 23
benachbarten Ecken 25 der Platten 21 eingreift. Die
von dem Querstab 24, den vorderen Stirnflächen 26 der
Platten 21 und der unteren Stirnfläche 27 der gekrümmten
Behälterwandung 22 gebildete Öffnung ist als Durchlaßöffnung 4 mit den Stegen 5 ausgebildet. Die Aufnahme
der Enden der Stege 5 erfolgt in dem Querstab 24 und/
oder der gekrümmten Behälterwandung 22 nahe deren unteren Stirnfläche 27        und ist auch hier nur schematisch   dargestellt. Eine detaillierte Beschreibung
ist weiter unten angegeben.

Die von dem Querstab 24, der oberen Stirnfläche 28 der Platten 21 und der oberen Stirnfläche 29 der gekrümmten Behälterwandung 22 gebildete Öffnung 30 stellt die Nachfüllöffnung dar, die durch die Klappe 20 beweglich verschließbar ist. Diese Klappe 20 ist um eine auf der Zylinderachse 23 liegenden Schwenkachse 31 verschwenkbar gelagert und schließt mit der gekrümmten Behälterwandung 22 und den Platten 21 sowie den Stegen 5 den Innenraum 16 zur Aufnahme der Wattekugeln 8 ein. Die Klappe 20 erstreckt sich soweit bis zur gekrümmten Behälterwandung 22, daß ein verbleibendes Spiel eine leichtgängige Bewegung zwischen der Klappe 20 und der gekrümmten Behälterwandung 22 erlaubt und gleichzeitig das Verklemmen von Wattekugeln 8 verhindert.

Die Schwenkachse 31 der Klappe 20 wird durch zwei Schrauben 32 gebildet, die durch miteinander fluchtende Bohrungen 33 in den Platten 21 hindurch in entsprechend angebrachte Gewindebohrungen 34 in der Klappe 20 eingeschraubt sind.

Im folgenden soll die Funktion der erfindungsgemäßen Vorrichtung zum Spenden von Wattekugeln kurz beschrieben werden.

Durch Abheben des oberen Teiles des zylindrisch ausgebildeten Aufnahmebehälters vom Stempel bzw. durch Schwenken der Klappe der als Trog gestalteten Vorrichtung nach außen, ist der Innenraum des Aufnahmebehälters frei zugänglich und kann mit Wattekugeln gefüllt werden. Der Aufsetz- bzw. der Schließvorgang werden in entsprechender Weise vorgenommen. Im Falle der zylindrisch ausgebildeten Vorrichtung ist zu erwähnen, daß bedingt durch die im Stempel angebrachten Nuten und die entsprechenden durch die Wandung des oberen Teiles des Aufnahmebehälters hindurchtretenden Schrauben, das Abheben und das Aufsetzen des oberen Teiles auf den Stempel nur in einer bestimmten Relativposition dieser beiden Teile möglich ist.

Das obere Teil wird nach dem Aufsetzen auf den Stempel gegenüber diesem verdreht und ist somit gegen ein unbeabsichtigtes Lösen gesichert.

Je nach Gewicht des oberen Teiles bzw. der Klappe des Aufnahmebehälters wird durch die Schwerkraft ein entsprechender Druck auf die Wattekugeln ausgeübt, wodurch diese in Richtung der Durchlaßöffnung und durch dieselbe hindurchgedrückt werden. Bei verfilzten Wattekugeln beispielsweise ist es möglich, daß die Gewichtskraft des oberen Teiles bzw. der Klappe nicht ausreicht, um den für das Hindruchtreten der Wattekugeln durch die Durchlaßöffnung notwendigen Druck zu erzeugen. In diesem Falle besteht eine einfache Maßnahme zur Druckerhöhung darin, daß der Benutzer die Klappe bzw. den oberen Teil des Aufnahmebehälters nach unten drückt.

Die parallele Anordnung der Stege und die im Querschnitt kreisförmige Ausbildung der Stege mit einem Durchmesser, der im wesentlichen dem Radius der Wattekugeln entspricht, erleichtert das Hindurchtreten derselben durch die Durchlaßöffnung. Die Stege dienen hier vorwiegend als Führungsmittel für die Wattekugeln, die sich infolge des auf sie ausgeübten Druckes an den gekrümmten Oberflächen der Stege nach außen abwälzen.

Im Falle der zylindrisch ausgebildeten Vorrichtung zum Spenden von Wattekugeln bewirkt ein einfaches Verdrehen des oberen Teiles gegenüber dem Stempel ein verbessertes Abwälzen der Wattekugeln an den Stegen und somit ein leichteres Hindurchtreten derselben durch die Durchlaßöffnung.

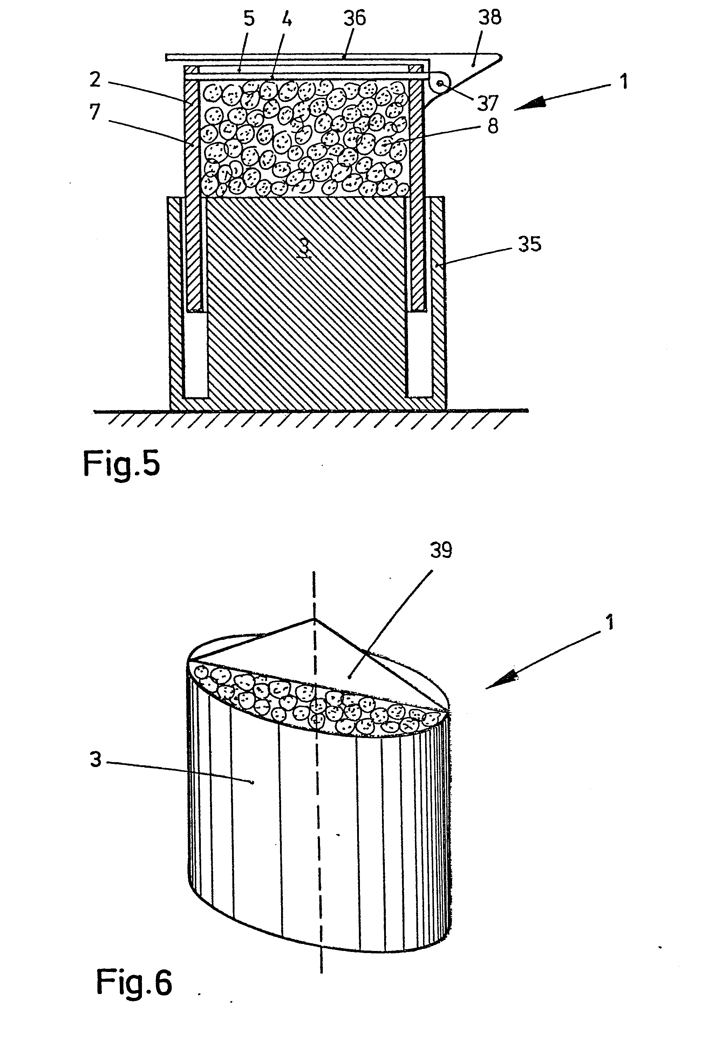

In Figur 5 ist eine dritte Ausführungsform der Erfindung gezeigt, die prinzipiell ähnlich ist zu der Ausführungsform der Figuren 1 und 2. Für gleiche oder äquivalente Teile werden deshalb gleiche Bezugszeichen verwendet. Nachfolgend wird nur noch auf die Unterschiede Bezug genommen.

Bei der Ausführungsform von Figur 5 ist der Stempel 3 umlaufend von einem äußeren Behälter 35 in Form einer Zylinderwandung umgeben, die im vorliegenden Fall mit ihm einstückig verbunden ist. Die Wandung hat den Zweck, für die Hubbewegung des oberen Teiles 2 als zusätzliche Führung zum Stempel 3 zu dienen. Gleichzeitig ermöglicht die Wandung eine bessere Handhabung des Aufnahmebehälters insgesamt. Will man nämlich denselben insgesamt hochheben, so ist es nämlich möglich, den Behälter 35 zu ergreifen. Hierzu kann auch mit der Hand der obere Teil 2 übergriffen werden.

Bei der Ausführungsform von Figur 5 ist zum Abschluß der Durchlaßöffnung 4 ein Deckel 36 vorgesehen, der mit Hilfe eines Scharniers 37 am oberen Teil angelenkt ist. Der Deckel dient zum Schutz gegen die Kontaminierung durch eine eventuell keimhaltige Atmosphäre. Er kann für die Benutzung des Aufnahmebehälters bzw. für die Entnahme der Wattekugeln 8 mit Hilfe der Handhabe 38 hochgeschwenkt werden.

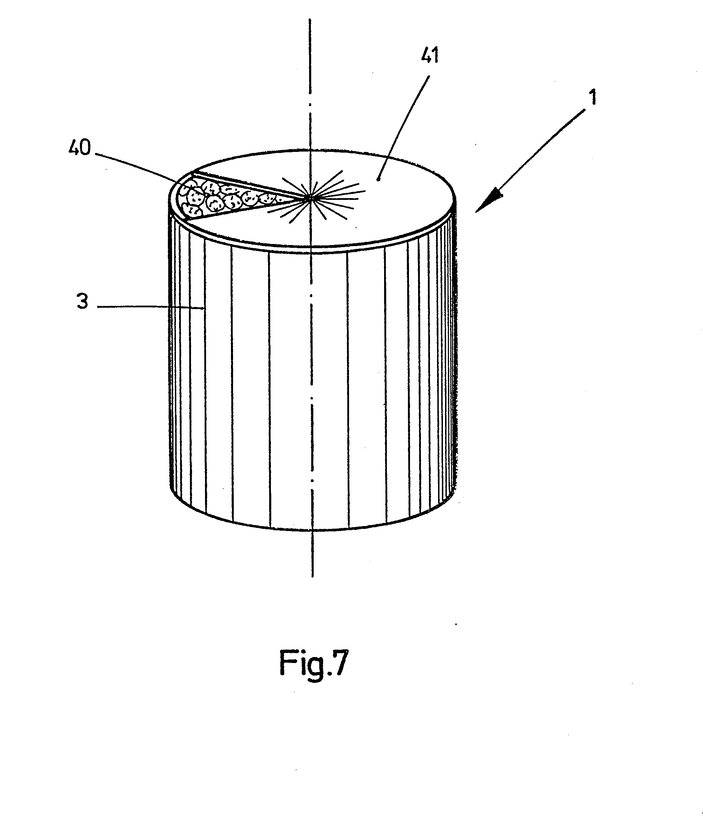

In den Figuren 6 und 7 sind zwei Möglichkeiten für Flügel dargestellt, nämlich einmal in Figur 6 ein Flügel in Form einer dreiecksförmigen, vertikal auf der Stempeloberfläche stehenden Platte 39 und einmal in Figur 7 in Form einer vertikal stehenden Verbindungsfläche 40 zu einer Spiralschrägfläche 41 des Stempels 3. Bei beiden Fällen dienen die vertikalen Flächen der Flügel 39 und 40 dazu, bei stehendem Stempel 3 und einer gleichzeitigen Verdrehung des Oberteils den Vorrat an Wattekugeln daran zu hindern, zusammen mit dem Oberteil zu rotieren. Durch das Hemmen der Verdrehbewegung der Wattekugeln will man erreichen, daß beim Verdrehen des Oberteiles 2 die Wattekugeln durch Berührung mit den Stegen 5 herausquirlen. Die Verdrehbewegung kann auf diese Weise das Nach-außen-Treten der Wattekugeln aus der Durchlaßöffnung erleichtern.

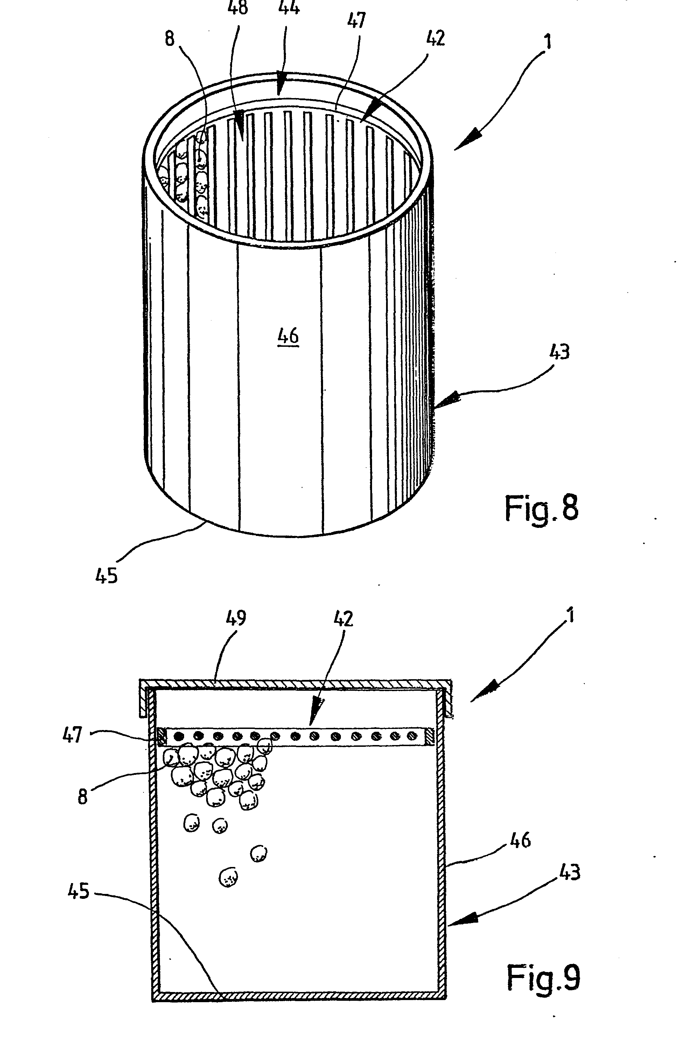

Die Figuren 8 und 9 zeigen als viertes Ausführungsbeispiel der Erfindung eine geradzylindrische Vorrichtung zum Spenden von Wattekugeln mit einem zweigeteilten Aufnahmebehälter 1, dessen erster Teil durch einen Einsatz 42 und dessen zweiter Teil durch ein Behältnis 43 dargestellt wird. Das Behältnis 43 weist die Form eines geraden hohlzylindrischen Körpers mit einer oberen Öffnung 44, einem dieser gegenüberliegenden Boden 45 und einer Wandung 46 auf. Es dient zur Aufnahme von Wattekugeln 8.

Der oberhalb der Wattekugeln 8 innerhalb des Behältnisses 43 in dessen Längsrichtung bewegbar angeordnete Einsatz 42 besteht aus einem kreisförmigen Ring 47, der die Durchlaßöffnung 48 für die Wattekugeln begrenzt. Diese Durchlaßöffnung 48 ist durch parallel angeordnete Stege 5 teilverschlossen, die einteilig mit dem Ring 47 des Einsatzes 42 verbunden sind. Die Stege 5 und die Lagerung der Stegenden sind auch hier nur schematisch dargestellt.

Eine detaillierte Beschreibung erfolgt weiter unten.

In Figur 9 ist das Behältnis 43 mit einem zylindrischen Deckel 49 verschlossen.

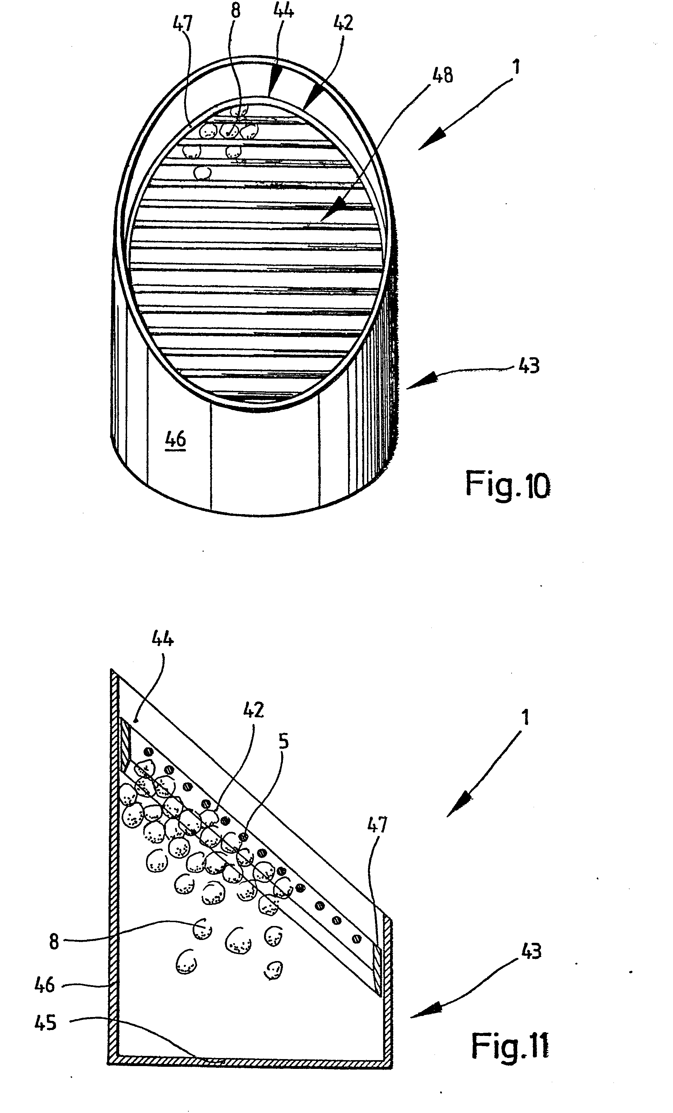

Die in den Figuren 10 und 11 als fünftes Ausführungsbeispiel der Erfindung dargestellte Vorrichtung zum Spenden von Wattekugeln entspricht im wesentlichen der in den Figuren 8 und 9 gezeigten Vorrichtung, jedoch mit den folgenden Unterschieden.

Um ein Verdrehen des Einsatzes 42 in dem Behältnis 43 zu verhindern, sind beide Teile in der Draufsicht ellipsenförmig ausgebildet. Die obere Begrenzungsfläche des Behältnisses 43, d. h. dessen obere Öffnung 44 bzw. eine durch dieselbe gelegte Ebene ist schräg unter einem von 90° abweichenden Winkel zur Längsachse des Behältnisses 43 verlaufend ausgebildet. Der Ring 47 und die Stege 5 des Einsatzes 42 verlaufen parallel zur oberen Öffnung 44.

Die Stege 5 weisen in diesem Ausführungsbeispiel im Querschnitt die Form eines Halbkreises mit in Richtung der Wattekugeln 8 zugewandten Rundung auf. Der Ring 47 des Einsatzes 42 ist zum Zwecke der besseren Führung durch ein sich in Richtung des Bodens 45 des Behältnisses 43 erstreckenden Flansch 50 verlängert. Die untere Kante dieses Flansches 50 läuft von innen nach außen keilförmig in eine Spitze aus, um bei der Bewegung des Einsatzes 42 nach unten ein Einklemmen der Wattekugeln 8 zwischen dem Flansch 50 und dem Behältnis 43 zu verhindern.

Die Funktion der in den Figuren 8 und 9 dargestellten Vorrichtung entspricht sinngemäß der Funktion der bereits auf Seite 21 ff. beschriebenen Vorrichtung gemäß den Figuren 1 bis 4. Gleiches gilt für die Funktion der in den Figuren 10 und 11 gezeigten Vorrichtung mit dem Unterschied, daß hier ein Verdrehen des Einsatzes innerhalb des Behältnisses durch die in der Draufsicht ellipsenförmige Ausbildung beider Teile unmöglich ist.

Die Entnahme der Wattekugeln wird dadurch erleichtert, daß die obere Öffnung des Behältnisses bzw. eine durch dieselbe gelegte Ebene sowie der Einsatz schräg unter einem Winkel zur Längsachse des Behältnisses verlaufend ausgebildet sind. Die zu entnehmenden Wattekugeln sind den Blicken eines sitzenden Benutzers beispielsweise leicht zugänglich und können von diesem auch bei größerem seitlichen Abstand ohne Abwinkeln der Hand leicht entnommen werden.

Natürlich kann der die ringförmige Begrenzung der Durchlaßöffnung des Einsatzes in Richtung der Wattekugeln verlängernde Flansch auch so ausgebildet sein, daß der Einsatz im Querschnitt sich in Richtung der Wattekugeln trichterförmig erweitert. Die ringförmige Begrenzung der Durchlaßöffnung des Einsatzes ist selbstverständlich nicht auf die Ringform beschränkt.
Auch ist es denkbar, die Durchlaßöffnung des Einsatzes anders als bisher beschrieben zu gestalten. So kann dieselbe ohne Stege ausgebildet sein und in der Draufsicht eine sternförmige Gestalt aufweisen. Es ist dadurch möglich, dieses Teil im Stanzverfahren besonders kostengünstig herzustellen.

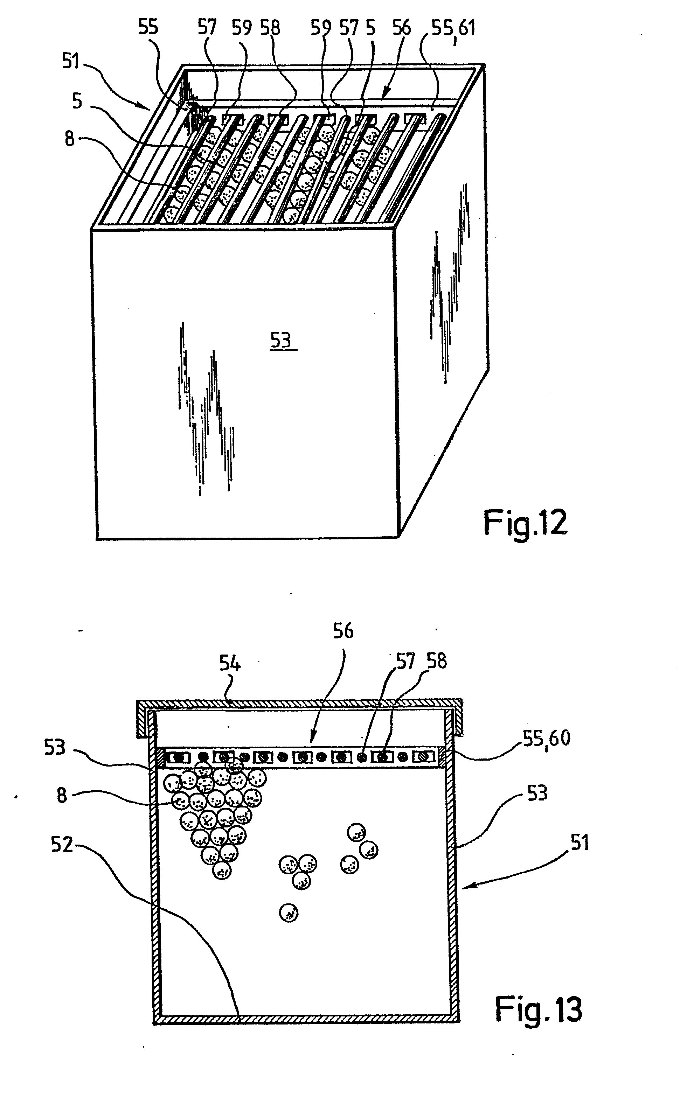

Ein sechstes Ausführungsbeispiel der erfindungsgemäßen Vorrichtung zum Spenden von Wattekugeln nach den Figuren 12 bis 14 umfaßt einen rechteckigen Behälter 51 zur Aufnahme von Wattekugeln 8 mit einem Boden 52 und vier Seitenwänden 53. Der Behälter 51 ist oben offen und kann, wie in Figur 13 dargestellt, durch einen Deckel 54 verschlossen sein und jede andere geeignete Form, beispielsweise die eines Zylinders, aufweisen. Ein den Seitenwänden 53 anliegender rahmenförmiger Einsatz 55, der einer dem Boden 52 parallele Durchlaßöffnung 56 für die Wattekugeln 8 begrenzt, ist verschiebbar im Behälter 51 angeordnet. Die Durchlaßöffnung 56 ist durch in einer Ebene parallel angeordnete elastische Stege 5 mit kreisrundem Querschnitt teilverschlossen.

Jeder elastische Steg 5 ist mit einem Stegende 57 im Einsatz 55 fest eingespannt. Das andere Stegende 58 ist in einer im Einsatz 55 ausgebildeten Nut 59 mit Spiel aufgenommen. Diese zur Durchlaßöffnung 56 hin offene Nut 59 verläuft in Umfangsrichtung des Einsatzes 55 und dient als Führung für das infolge der Elastizität des Steges 5 bewegliche Stegende 58. Jedem elastischen Steg 5 bzw. Stegende 58 ist eine Nut 59 zugeordnet, deren Länge die Bewegungsamplitude des Stegendes 58 bestimmt. Die Stegenden 57 bzw. 58 benachbarter elastischer Stege 5 sind an gegenüberliegenden Rahmenteilen 60 und 61 des Einsatzes 55 eingespannt bzw. beweglich gelagert. Solange keine Wattekugeln 8 entnommen werden, befinden sich die elastischen Stege 5 in der Ruhestellung, d. h. sie sind geradlinig und mit ihren Stegenden 58 in der Längsmitte der jeweiligen Nuten 59 angeordnet - siehe Steg 1 bis 5 und 8 bis 11 von links in Figuren 12 und 14 - . In Ruhestellung ist der Stegabstand kleiner als der Durchmesser der Wattekugeln.

Bei der Entnahme einer Wattekugel 8 zwischen zwei benachbarten Stegen hindurch - siehe Steg 6 und 7 von links in Figuren 12 und 14 - erfährt dieselbe eine Verformung. Die dabei auftretenden Verformungskräfte werden von den Stegen aufgenommen und bewirken je nach deren Biegesteifigkeit ein Ausweichen derselben gegenüber der Wattekugel. Das Maß, um welches die Stege ausweichen, ist abhängig vom Angriffspunkt der Wattekugel. Wird dieselbe in der Stegmitte zwischen den beiden Stegenden 57 und 58 entnommen, so weichen beide elastischen Stege 5 um das gleiche Maß so weit aus, daß die Wattekugel ungehindert hindurchtreten kann. Wird die Wattekugel jedoch außerhalb der Stegmitte entnommen, so weicht der elastische Steg 5, dessen fest eingespanntes Stegende 57 weiter von der Wattekugel 8 entfernt ist, um ein größeres Maß als der andere elastische Steg 5 aus. Unabhängig vom Angriffspunkt der Wattekugel 8 ist die Abstandsvergrößerung zwischen benachbarten elastischen Stegen 5 infolge deren Ausweichbewegung konstant. Die Länge der Nut 59 ist hierbei so zu bemessen, daß das bewegliche Stegende 58 seine maximale Ausweichbewegung ohne Behinderung durchführen kann.

Die Entnahme der Wattekugeln 8 wird dadurch unterstützt, daß sie an den Stegen 5 infolge deren kreisrunder Querschnitte abrollen können. Eine weitere Unterstützung der Entnahme der Wattekugeln 8 ergibt sich dann, wenn die Stege 5 mit ihren Stegenden 57 nicht fest eingespannt, sondern derart gelagert sind, daß sie eine Drehbewegung um die Stegachse durchführen können.

Die durch die Nuten 59 erreichte Beschränkung der Ausweichbewegung der elastischen Stege 5 auf die Durchlaßlichen der in den Figuren 12 bis 14 dargestellten Vorrichtung, jedoch mit einer unterschiedlichen Steglagerung. Der Unterschied besteht darin, daß die beweglichen Stegenden 58 der elastischen Stege 5 in jeweils einer Nut 62 im Rahmen 61 des Einsatzes 55 geführt sind. Diese Nuten 62 erstrecken sich in Verschieberichtung des Einsatzes 55, d. h. normal zu den Nuten 59 im gegenüberliegenden Rahmenteil 60 des Einsatzes 55. Dies bedeutet, daß die Ausweichbewegung der in den gegenüberliegenden Rahmenteilen 60 und 61 des Einsatzes 55 beweglich gelagerten Stegenden 58 benachbarter elastischer Stege 5 auf zwei zueinander normal angeordneten Ebenen beschränkt ist. Im Rahmenteil 60 erfolgt die Ausweichbewegung infolge der Führung durch die Nuten 59 in der Ebene der Durchlaßöffnung 56 und im Rahmenteil 61 infolge der Führung durch die Nuten 62 in einer zur Ebene der Durchlaßöffnung 56 normalen Ebene. Die Nuten 62 sind derart angeordnet, daß in der Ruhestellung der zugeordneten Stege 5 deren Stegenden 58 der unteren Schmalseite der Stege 62 aufliegen. Mit anderen Worten, die Stegenden 58 können nur nach oben ausweichen. Eine Bewegung in Richtung zum Behälterinneren ist ausgeschlossen.

Auch hier sind die Stege mit kreisrundem Querschnitt versehen und können ebenfalls drehbar gelagert sein. Zur Vereinfachung der Konstruktion ist es auch hier möglich, die Funktion der Nuten 59 und 62 durch hochkant bzw. flachkant angeordnete Stege mit rechteckigem Querschnitt zu ersetzen. Nicht drehbar gelagerte kreisrunde oder rechteckige Stege können auch über ihre Stegenden 57 einteilig mit dem Einsatz 55 verbunden sein.

Im Unterschied zu den vorhergehenden Ausführungsbeispielen zeigt das in Figur 18 dargestellte achte Ausführungsbeispiel elastische Stege 5, deren Stegenden 58 frei beweglich sind. Zu diesem Zwecke ist die Länge der Stege 5 kürzer als der Abstand der gegenüberliegenden Rahmenteile 60 und 61 des Einsatzes 55. Die Stege 5 können demzufolge während der Entnahme von Wattekugeln 8 je nach deren Angriffspunkt in jeder möglichen sich entlang der Stegachse erstreckenden Ebene ausweichen.

Auch hier treffen die für die beiden vorhergehenden Ausführungsbeispiele gemachten Ausführungen hinsichtlich der Einspannung der Stegenden 57 benachbarter Stege 5 an den gegenüberliegenden Rahmenteilen 60 und 61 sowie hinsichtlich der Alternativlösungen zu.

Bei allen vorstehend beschriebenen Ausführungsbeispielen ist es möglich, daß die Stege in mehreren Ebenen und gegebenenfalls gekreuzt zueinander angeordnet sind.

GRÜNECKER, KINKELDEY, STOCKMAIR & PARTNER

PATENTANWÄLTE
EUROPEAN PATENT ATTORNEYS

A. GRÜNECKER, DIPL.-ING
DR. H. KINKELDEY, DIPL.-ING
DR. W. STOCKMAIR, DIPL.-ING, A.E.E (CALTECH)
DR. K. SCHUMANN, DIPL.-PHYS
P. H. JAKOB, DIPL.-ING
DR. G. BEZOLD, DIPL.-CHEM
W. MEISTER, DIPL.-ING
H. HILGERS, DIPL.-ING
DR. H. MEYER-PLATH, DIPL.-ING

8000 MÜNCHEN 22
MAXIMILIANSTRASSE 43

Roescheisen GmbH & Co.                    EP 1069 - 806

Scheffeltgasse 3

7900 Ulm


Vorrichtung zum Spenden von Wattekugeln


P a t e n t a n s p r ü c h e


1. Vorrichtung zum Spenden von Wattekugeln mit einem zweigeteilten Aufnahmebehälter für die Wattekugeln, dessen erster Teil mit einer durch Stege teilverschlossenen Durchlaßöffnung und dessen zweiter Teil mit einer Druckfläche bzw. einem Boden des Aufnahmebehälters versehen ist, wobei die Wattekugeln mittels einer Relativbewegung der beiden Teile des Aufnahmebehälters durch die Durchlaßöffnung hindurch bewegt werden, dadurch g e k e n n z e i c h n e t , daß die Relativbewegung der beiden Teile (2, 3, 35; 19, 20; 42, 43; 55, 51) des Aufnahmebehälters (1) durch die Schwerkraft erreicht wird und der Durchmesser der Stege in der Ebene der Durchlaßöffnung (4, 48, 56) im wesentlichem dem Radius der Wattekugeln (8) entspricht.

2. Vorrichtung nach Anspruch 1, dadurch g e k e n n - z e i c h n e t , daß der erste, die Durchlaßöffnung (4) aufweisende Teil des Aufnahmebehälters (1) als oberer Teil (2), mit einer der Durchlaßöffnung (4) gegenüberliegenden Bodenöffnung (9) versehen, auf dem als Stempel (3) ausgebildeten zweiten unteren Teil des

3. Vorrichtung nach Anspruch 1 oder 2, dadurch g e k e n n - z e i c h n e t , daß zumindest ein Teil der Stege elastisch ausgebildet ist.

4. Vorrichtung nach Anspruch 3, dadurch g e k e n n z e i c h - n e t , daß die elastischen Stege (5) mit jeweils wenigstens einem Stegende (58) gegenüber den durchtretenden Wattekugeln (8) elastisch ausweichend in wenigstens einer die Stegachse einschließenden Ebene gelagert sind.

5. Vorrichtung nach Anspruch 3, dadurch g e k e n n - z e i c h n e t , daß die elastischen Stege (5) an beiden Stegenden (58) in jeweils einer sich entlang der Stegachse erstreckenden Ebene gegenüber den durchtretenden Wattekugeln (8) ausweichend gelagert sind, wobei die eine Ebene gegenüber der anderen um etwa 90° gedreht ist.

6. Vorrichtung nach Anspruch 3, dadurch g e k e n n - z e i c h n e t , daß die elastischen Stege (5) an jeweils einem Stegende (57) in der Art eines Freiträgers fest gelagert und an dem anderen Stegende (58) in einer sich entlang der Stegachse erstreckenden Ebene gegenüber den durchtretenden Wattekugeln (8) ausweichend gelagert ist.

7. Vorrichtung nach wenigstens einem der Ansprüche 3 bis 6, dadurch g e k e n n z e i c h n e t , daß zumindest die elastischen Stege (5) einen im wesentlichen kreisförmigen Querschnitt aufweisen.

8. Vorrichtung nach wenigstens einem der Ansprüche 3 bis 7, dadurch g e k e n n z e i c h n e t , daß zumindest die elastischen Stege (5) um ihre Stegachse drehbar sind.

9. Vorrichtung nach wenigstens einem der Ansprüche 3, 4,7 und 8, dadurch g e k e n n z e i c h n e t , daß die elastischen Stege (5) in der Art eines Freiträgers an lediglich einem Stegende (57) fest gelagert sind.

10. Vorrichtung nach wenigstens einem der Ansprüche 3,4 und 6 bis 9, dadurch g e k e n n z e i c h n e t , daß zumindest die elastischen Stege (5) mit ihren jeweils in der Art eines Freiträgers gelagerten Stegende (57) einteilig mit dem ersten Teil des Aufnahmebehälters (1) verbunden sind.

11. Vorrichtung nach wenigstens einem der Ansprüche 4 – 6 und 8 – 10,dadurch g e k e n n z e i c h n e t , daß zumindest die elastischen Stege (5) einen rechteckigen Querschnitt aufweisen, dessen Breitseite in die Ausweichrichtung der elastischen Stege (5) weist.

12. Vorrichtung nach wenigstens einem der Ansprüche 4 bis 11, dadurch g e k e n n z e i c h n e t , daß einander benachbarte elastische Stege (5) an jeweils entgegengesetzten Stegenden (57) in der Art eines Freiträgers fest gelagert sind.

13. Vorrichtung nach wenigstens einem der Ansprüche 3 bis 12, dadurch g e k e n n z e i c h n e t , daß jeweils ein elastischer Steg (5) abwechselnd mit einem weniger elastischen und/oder mit einem an beiden Stegenden fest gelagerten Steg angeordnet sind.

14. Vorrichtung nach wenigstens einem der Ansprüche 1 bis 13, dadurch g e k e n n z e i c h n e t , daß die Stege im wesentlichen parallel angeordnet sind.

15. Vorrichtung nach wenigstens einem der Ansprüche 1 bis 14, dadurch g e k e n n z e i c h n e t , daß die Stege als gerade Stege mit glatter Oberfläche ausgebildet sind.

16. Vorrichtung nach wenigstens einem der Ansprüche 1 bis 15, dadurch g e k e n n z e i c h n e t , daß die Stege im wesentlichen in einer Ebene angeordnet sind.

17. Vorrichtung nach wenigstens einem der Ansprüche 2 bis 16, dadurch g e k e n n z e i c h n e t , daß die obere Begrenzungsfläche des Stempels (3) als eine im wesentlichen ebene und senkrecht zur Längsachse des Aufnahmebehälters (1) angeordnete Stempelfläche (13) ausgebildet ist.

18. Vorrichtung nach wenigstens einem der Ansprüche 1 bis 17, dadurch g e k e n n z e i c h n e t , daß der erste Teil (2) und der zweite Teil (3) zylindrisch ausgebildet sind.

19. Vorrichtung nach wenigstens einem der Ansprüche 2 bis 18, dadurch g e k e n n z e i c h n e t , daß der Stempel (3) einen der Stempelfläche (13) zugeordneten, durch mindestens eine im wesentlichen vertikale Nut (15) durchbrochenen umlaufenden Bund (12) und der erste Teil (2) des Aufnahmebehälters (1) einen in seinen Innenraum (16) hineinragenden, der Nut (15) entsprechenden Vorsprung (17) aufweist.

20. Vorrichtung nach Anspruch 19, dadurch g e - k e n n z e i c h n e t , daß die Nut (15) eine geringere Tiefe aufweist als der Bundstärke entspricht und der der Nut (15) entsprechende Vorsprung (17) in seiner Eindringtiefe verstellbar ausgebildet ist.

21. Vorrichtung nach wenigstens einem der Ansprüche 2 bis 20, dadurch g e k e n n z e i c h n e t , daß mindestens ein Flügel im wesentlichen senkrecht an der Stempelfläche (13) angebracht ist.

22. Vorrichtung nach Anspruch 21, dadurch g e - k e n n z e i c h n e t , daß der Flügel mit einem im wesentlichen dreieckigen, mit der Basis zur Stempelfläche (13) weisenden Querschnitt an derselben angebracht ist.

23. Vorrichtung nach Anspruch 21 oder 22, dadurch g e k e n n z e i c h n e t , daß die obere Begrenzungsfläche des Flügels als eine sich über die gesamte Stempelfläche (13) erstreckende, in Umfangsrichtung von der Oberkante bis zur Unterkante des Flügels abfallenden Schräge ausgebildet ist.

24. Vorrichtung nach wenigstens einem der Ansprüche 1 bis 23, dadurch g e k e n n z e i c h n e t , daß der Aufnahmebehälter (1) insgesamt von einem mit einer oberen Öffnung versehenen äußeren Behälter (35) umschlossen ist.

25. Vorrichtung nach wenigstens einem der Ansprüche 1 bis 24, dadurch g e k e n n z e i c h n e t , daß

der erste Teil (2) des Aufnahmebehälters (1) als mit Wattekugeln (8) gefüllte und mittels Folien an seiner Bodenöffnung (9) und der Durchlaßöffnung (4) luftdicht verschlossene, auswechselbare Einsatzpackung ausgebildet ist.

26. Vorrichtung nach wenigstens einem der Ansprüche 1 und 3 bis 16, dadurch gekennzeichnet , daß der Aufnahmebehälter (1) im wesentlichen als Trog (19) ausgebildet ist, der die Form eines Zylinder-Viertels aufweist, wobei die dem einen Zylinder-Radius folgende erste Behälterwandung als erfindungsgemäße Durchlaßöffnung (4) und die dem anderen Zylinder-Radius folgende zweite Behälterwandung als verschwenkbare, bis zur dritten, der Mantelfläche des Zylinder-Viertels folgenden gekrümmten Behälterwandung (22) reichende Klappe (20) ausgebildet ist, deren Schwenkachse (31) parallel zu und nahe der Zylinderachse (23) angeordnet ist.

27. Vorrichtung nach wenigstens einem der Ansprüche 1 bis 26, dadurch gekennzeichnet , daß der Aufnahmebehälter (1) in mindestens zwei Kammern zur Aufnahme von Wattekugeln (8) mit unterschiedlichen Durchmessern aufgeteilt ist.

28. Vorrichtung nach wenigstens einem der Ansprüche 1 bis 27, dadurch gekennzeichnet , daß der Durchmesser der Stege (5) kleiner ist als der Radius der Wattekugeln (8).

29. Vorrichtung nach wenigstens einem der Ansprüche 1 und 3 bis 16, 18, 24, 27 und 28, dadurch gekenn-

z e i c h n e t , daß der erste Teil (42, 55) des Aufnahmebehälters (1) als ein innerhalb des zweiten Teiles (43, 51) relativ zu diesem bewegbarer Einsatz (42, 55) ausgebildet ist, wobei der zweite Teil (43, 51) zumindest Führungsflächen für den ersten Teil (42, 55) und eine obere Öffnung (44) zum Entnehmen der Wattekugeln (8) aufweist.

30. Vorrichtung nach Anspruch 29, dadurch g e - k e n n z e i c h n e t , daß der zweite Teil (43, 51) des Aufnahmebehälters (1) als ein Behältnis (43, 51) für die Wattekugeln (8) mit der oberen Öffnung (44), mit einem Boden (45,52) und mit Wandungen (46, 53), die zur Führung des ersten Teiles (42, 55) dienen, ausgebildet ist.

31. Vorrichtung nach Anspruch 29 oder 30, dadurch g e k e n n z e i c h n e t , daß die obere Öffnung (44) von den Führungsflächen bzw. Wandungen (46) direkt begrenzbar ausgebildet ist.

32. Vorrichtung nach wenigstens einem der Ansprüche 29 bis 31, dadurch g e k e n n z e i c h n e t , daß die obere Öffnung (44) des zweiten Teiles (43) des Aufnahmebehälters (1) bzw. eine durch dieselbe gelegte Ebene sowie der erste Teil (42) schräg unter einem von 90° abweichenden Winkel zur Längsachse des Aufnahmebehälters (1) verlaufend ausgebildet sind.

33. Vorrichtung nach wenigstens einem der Ansprüche 1 bis 30, dadurch g e k e n n z e i c h n e t , daß der erste Teil (19, 55) und der zweite Teil (20, 51) des Aufnahmebehälters (1) abweichend von der zylindrischen Form ausgebildet sind.

34. Vorrichtung nach wenigstens einem der Ansprüche 29 bis 33, dadurch g e k e n n z e i c h n e t , daß der erste Teil des Aufnahmebehälters mit in Richtung des Bodens und der Wandungen des zweiten Teils des Aufnahmebehälters verlaufenden Schrägflächen zur Führung der Wattekugeln zur Durchlaßöffnung ausgebildet ist.

**Fig.1**

**Fig. 2**

Fig. 3

Fig. 4

0093315

Fig.5

Fig.6

Fig.7

Fig.8

Fig.9

0093315

48

0093315

47  8  44  42

48

1

43

46

Fig.10

44

42

5

1

47

8

43

46

45

Fig.11

Fig.12

Fig.13

0093315

0093315

Fig. 14

Fig. 15

Fig. 16

Fig. 17

Fig. 18